# EUROPEAN PATENT APPLICATION

(11) **EP 4 721 651 A1**
(43) Date of publication of application: **08.04.2026**
(21) Application number: 24204930.2
(22) Date of filing: 07.10.2024
(51) Int. Cl.: A61B 5/0205, A61B 5/021, A61B 5/024, A61B 5/11, A61B 5/352, A61B 5/366, A61B 5/00

(54) **ELECTROCARDIOGRAPHIC CARDIAC MONITORING**

(71) Applicant: Koninklijke Philips N.V., 5656 AG Eindhoven (NL)
(72) Inventor: BONOMI, Alberto Giovanni, Eindhoven (NL)
(74) Representative: Philips Intellectual Property & Standards

(57) **Abstract**

Proposed concepts aim to provide methods and systems pertaining to assessing the quality of an ECG signal. In particular, embodiments aim to provide a method for automatically determining a signal quality value of an ECG signal based on a fiducial point of the ECG signal that has been identified using a non-ECG cardiac signal.

## Description

### FIELD OF THE INVENTION

The invention relates to the field of cardiac monitoring, and more specifically to the field of electrocardiogram monitoring of cardiac systems.

### BACKGROUND OF THE INVENTION

Electrocardiograms (ECGs) are routinely used to monitor cardiac electrical activity. During an ECG, electrode sensors are placed on the skin to measure small scale electrical signals that originate in the heart. ECG measurements are incorporated into numerous care pathways and are beneficial for the diagnosis of cardiac diseases and the identification of cardiac anomalies. In clinical practice, multiple ECG signals may be obtained during a single ECG examination. Typical ECG monitors involve multiple ECG channels (or leads) which are each associated with a different ECG sensor placed on the patient's skin and which each have their own associated ECG signal.

There are many ways in which ECG signals may be used for patient monitoring, and these different methods may have different ECG channel configuration requirements. Cardiac diagnosis typically requires 12-lead ECG recordings, or 1-3 ECG leads Holter monitoring. In critical care and telemetry wards patients are typically monitored using 4 ECG lead systems for real-time arrythmia detection and patient surveillance.

Noise is often present in ECG signals as the ECG comprises small scale electrical impulses that may be easily affected by patient movement or by motion of the ECG wires. Different leads of an ECG monitor may suffer different levels of noise and therefore the different ECG signals obtained during an ECG exam may vary in their quality. In addition, physiological characteristics of the patient and the positioning of the electrodes on the body may generate ECG waveforms that are difficult to interpret, despite the absence of noise.

ECG waveforms are interpreted by clinical experts who visually inspect the waveform. Artificial intelligence and other interpretation algorithms may also be used to streamline expert review of ECG signals and provide automatic identification of cardiac events for alarming purposes. The accuracy of such interpretation algorithms strongly depends on the quality of the ECG waveform. It may therefore be beneficial to have an automatic and standardized way of determining the signal quality of different ECG signals to ensure the best quality data is used for signal interpretation, in particular automatic signal interpretation.

### SUMMARY OF THE INVENTION

The invention is defined by the claims.

According to examples in accordance with an aspect of the invention, there is provided a computer-implemented method for assessing the quality of an ECG signal.

The method comprises: obtaining an ECG signal and a non-ECG cardiac signal for use in monitoring the cardiac activity of a subject; identifying a fiducial point of the ECG signal based on the non-ECG cardiac signal; and determining a signal quality value of the ECG signal based on the identified fiducial point of the ECG signal.

Proposed concepts thus aim to provide schemes, solutions, concepts, designs, methods, and systems pertaining to assessing the quality of an ECG signal. In particular, embodiments aim to provide a method for automatically determining a signal quality value of an ECG signal based on a fiducial point of the ECG signal that has been identified using a non-ECG cardiac signal.

Therefore, in the proposed invention, the signal quality of an ECG signal is assessed in an automated and standardized way based on an identified fiducial point in the ECG. ECG fiducial points are points or regions of an ECG signal that correspond to key cardiac events within the cardiac cycle. For example, the fiducial point may describe a portion of the ECG signal corresponding to a time interval in which a cardiac event (such as a particular type of heartbeat or a particular point within a heartbeat) takes place. Analysis of ECG fiducial points may be used for identifying certain cardiac disorders and arrythmias and therefore may be relevant for assessing the usefulness of an ECG signal for cardiac monitoring and diagnosis.

In particular, in the proposed invention, fiducial points are identified and extracted from the ECG signal based on a non-ECG cardiac signal. ECG monitoring is a highly sensitive cardiac monitoring technique that captures a subject's cardiac activity throughout the cardiac cycle. Other cardiac monitoring techniques, for example pulsatile monitoring techniques such as photoplethysmogram (PPG) monitoring or arterial blood pressure (ABP) monitoring, are less sensitive to the occurrence of certain cardiac events. Therefore, certain cardiac events, such as ectopic cardiac beats or premature ventricular contractions, are not clearly visible in such non-ECG cardiac waveforms. By identifying fiducial points in an ECG signal based on a non-ECG cardiac signal, only fiducial points relating to certain cardiac events that are clearly discernible in the non-ECG cardiac signal are identified. This is advantageous as signal quality values based on fiducial points that are not discernible from non-ECG waveforms, such as premature ventricular contractions, may not provide a good indication of the usefulness of the signal for automatic interpretation and visualization, as these regions of the signal are not used for these processes.

Hence, the proposed invention leverages a non-ECG cardiac signal to identify fiducial points in an ECG signal that are relevant for the assessment of ECG signal quality. A signal quality value can then be determined for the ECG signal based on the identified fiducial point. The determined signal quality value may then be presented to a clinician to aid with manual interpretation of ECG waveforms or may be used to automatically select ECG signals or portions of ECG signals for further processing and display.

Ultimately, an improved method for assessing the quality of an ECG signal for monitoring the cardiac activity of a subject may be provided by the proposed concept(s).

In some embodiments, identifying a fiducial point of the ECG signal based on the non-ECG cardiac signal may comprise: processing the non-ECG cardiac signal with a cardiac event detection model to identify a portion of the non-ECG signal associated with a cardiac event of the subject; identifying a portion of the ECG signal that corresponds to the identified portion of the non-ECG cardiac signal; and identifying the fiducial point of the ECG signal based on the identified portion of the ECG signal. This may provide an accurate way of detecting cardiac events associated with relevant fiducial points in the ECG signal. Therefore, this embodiment may result in a more accurate assessment of the quality of the ECG signal and its usefulness for monitoring the cardiac activity of the subject.

In some embodiments, the cardiac event of the subject may comprise a heartbeat corresponding to the systolic phase of a cardiac cycle of the subject. These heart beats may provide a particularly accurate indication of the signal quality and its suitability for automatic arrythmia detection.

In some embodiments, identifying a portion of the ECG signal may comprise performing a time calibration process to synchronize the ECG signal and the non-ECG cardiac signal. A time delay may exist between signals captured via ECG and non-ECG monitoring techniques. Performing a time calibration process may provide an easy way to account for this delay and thereby provide accurate fiducial point identification.

In some embodiments, the fiducial point may comprise at least one of: a time interval corresponding to a heartbeat of the subject; an onset time of a heartbeat of the subject; an offset time of a heartbeat of the subject; and a time interval corresponding to a cardiac event. Each of these fiducial points may be represented in both ECG and non-ECG cardiac signals and have importance for cardiac monitoring functionalities such as arrythmia detection. The use of such fiducial points in the assessment of ECG signal quality may therefore improve how accurately the determined signal quality value reflects the potential usefulness of the ECG signal for cardiac monitoring.

In some embodiments, determining the signal quality value of the ECG signal may comprise: determining a value of a signal quality indicator based on a portion of the ECG signal associated with the identified fiducial point of the ECG signal; and determining the signal quality value based on the value of the signal quality indicator. Certain portions and properties of the ECG signal, known as signal quality indicators, may be particularly indicative of how successful automatic interpretation of the ECG signal is likely to be. Therefore, basing the determination of the signal quality value on the value of such a signal quality indicator may result in a more accurate evaluation of ECG signal quality.

In some embodiments, the signal quality indicator may comprise at least one of: an amplitude of a QRS complex; an amplitude difference between a QRS complex and a surrounding waveform of the ECG signal; a level of high frequency noise; a level of low frequency noise; a slope of a QRS complex; an area of a QRS complex; and a QRS complex polarity. Each of these quantities may act as a good metric for the quality of the ECG signal.

In some embodiments, the non-ECG cardiac signal may comprise at least one of: a PPG signal; an arterial blood pressure signal; a peripheral blood pressure signal; a ballisto-cardiograph signal; and a seismo-cardiograph signal. Each of these non-ECG cardiac signals may be easily obtained via conventional/known cardiac monitoring techniques and may permit the identification of suitable fiducial points of the ECG signal.

In some embodiments, the signal quality value may describe the suitability of the ECG signal for use in arrhythmia detection. Automatic arrythmia detection may be particularly sensitive to signal quality and therefore a signal quality value describing the suitability of the ECG signal for this use may be of particular importance.

In some embodiments, the signal quality value describes the suitability of the ECG signal for monitoring the cardiac activity of the subject during a predetermined time period. The quality of an ECG signal may vary over time as the various sources of noise affecting the signal vary over time. Thus, a time period specific signal quality value may provide a more accurate assessment of signal quality.

According to another aspect of the present invention there is provided a method for selecting ECG signals for use in monitoring the cardiac activity of a subject. The method comprises: obtaining a plurality of ECG signals for use in monitoring the cardiac activity of a subject; for each of the plurality of ECG signals determining a signal quality value of the ECG signal using any of the methods described above; and selecting an ECG signal from the plurality of ECG signals for use in monitoring the subject based on the determined signal quality values of each of the plurality of ECG signals.

According to another aspect of the present invention there is provided a computer program comprising computer code means adapted, when said computer program is run on a computer, to implement any of the above described methods.

According to yet another aspect of the proposed invention there is provided an ECG quality assessment system comprising: a data acquisition module configured to obtain an ECG signal and a non-ECG cardiac signal for use in monitoring the cardiac activity of a subject; and a processing arrangement configured to: identify a fiducial point of the ECG signal based on the non-ECG cardiac signal; and determine a signal quality value of the ECG signal based on the identified fiducial point of the ECG signal.

The ECG quality assessment system described above may in some embodiments be integrated into a patient monitoring system comprising: an ECG sensor configured to acquire an ECG signal; and a non-ECG signal configured to acquire a non-ECG cardiac signal.

Embodiments may be employed in combination with conventional/existing patient monitoring methods and systems. In this way, embodiments may integrate into legacy systems to improve and/or extend their functionality and capabilities. An improved ECG cardiac monitoring system that automatically assesses the quality of ECG signals for use in monitoring the cardiac activity of a subject may therefore be provided by proposed embodiments.

Thus, there may be proposed concepts for assessing the quality of ECG signals based on fiducial points identified using a non-ECG cardiac signal. These and other aspects of the invention will be apparent from an elucidated with reference to embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for assessing the quality of an ECG signal according to a proposed embodiment;
Fig. 2 depicts a visual representation of an assessment of signal quality based solely on an ECG lead signal;
Fig. 3 depicts a visual representation of an assessment of signal quality according to a proposed embodiment;
Fig. 4 is a simplified flow diagram of a method for selecting ECG signals for use in monitoring the cardiac activity of a subject according to an aspect of the present invention;
Fig. 5 is a simplified block diagram of a patient monitoring system according to another aspect of the present invention; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for the purpose of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes, and/or solutions pertaining to assessing the quality of ECG signals. According to proposed concepts, a number of possible solutions may be implemented separately or jointly. That is, although these possible solutions may be described below separately, two or more of these possible solutions may be implemented in one combination or another.

Embodiments of the proposed invention aim to provide a method for automatically assessing the quality of an ECG signal. This quality assessment is based on fiducial points in the ECG signal that have been identified using a non-ECG cardiac signal. This may advantageously provide a more reliable assessment of ECG signal quality.

By way of summary, the following steps outline a process according to the proposed concept(s):
Step 1: obtain ECG and non-ECG cardiac signals describing a subject's cardiac activity. The non-ECG cardiac signal may take many different forms. For example, the non-ECG cardiac signal may be a pulsatile signal such as a photoplethysmogram (PPG) signal or an arterial blood pressure signal (ABP) or a mechanical signal such as a seismocardiogram (SCG) signal or a ballistocardiogram signal.
Step 2: identify fiducial points of the ECG signal based on the non-ECG signal. Fiducial point is a term used in reference to ECG signals to describe the location of particular cardiac events within the ECG signal and their duration. In this step, information within the non-ECG signal is used to identify the location of fiducial points in the ECG signal.
Step 3: determine a signal quality value of the ECG from the identified fiducial points.

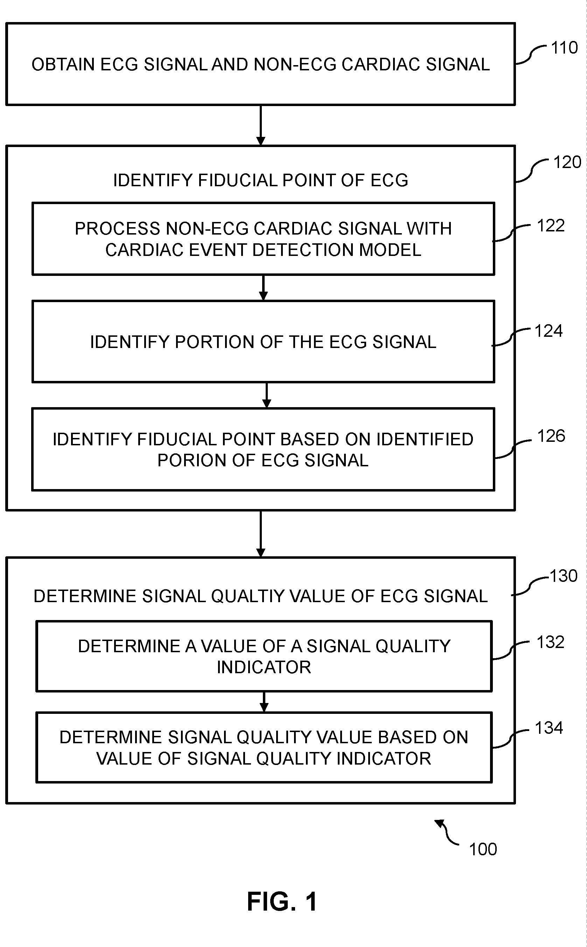

Referring now to Fig. 1, there is depicted a simplified flow diagram of a method 100 for assessing the quality of an ECG signal according to a proposed embodiment.

The method commences with a step 110 comprising obtaining an ECG signal and a non-ECG cardiac signal for use in monitoring the cardiac activity of a subject. In this exemplary embodiment the non-ECG cardiac signal is a PPG signal and both the non-ECG cardiac signal, and the ECG signal are obtained via a muti-parameter patient monitoring system.

The method then proceeds to a step 120 comprising identifying a fiducial point of the ECG signal based on the non-ECG cardiac signal. In this exemplary embodiment, step 120 comprises sub-steps 122, 124, and 126.

In step 122, the non-ECG cardiac signal is processed with a cardiac event detection model to identify a portion of the non-ECG signal associated with a cardiac event of the subject. Various algorithms suitable for cardiac event detection may be used in this step to identify signal features (i.e., characteristic amplitude variations and waveform shapes) that indicate the occurrence of a specific type of cardiac event. In this example, the cardiac event of the subject comprises a heartbeat corresponding to the systolic phase of a cardiac cycle of the subject.

Step 124 comprises identifying a portion of the ECG signal that corresponds to the identified portion of the non-ECG cardiac signal. In this embodiment, this step further comprises performing a time calibration process to synchronize the ECG signal and the non-ECG cardiac signal. Time delays may exist between different cardiac signals due to the physiological delay between the electrical activity and the hemodynamic activity of the cardiovascular system, or due to latency effects associated with signal acquisition and processing. In this step optimal time alignment between the ECG signal and the PPG signal is achieved by removing the arrive time delay in the beat time between electrical and pulsatile signals. This step could be carried out for each individual cardiac beat or collectively for a group of cardiac beats within a certain processing time window. The portion of the ECG signal corresponding to the identified portion of the cardiac signal can then easily be identified.

In step 126, the fiducial point of the ECG signal is then identified based on the identified portion of the ECG signal. In this example, the fiducial point comprises a time interval corresponding to a heartbeat of the subject identified in the non-ECG cardiac signal and therefore the fiducial point comprises the identified portion of the ECG signal in this case.

Once the fiducial point of the ECG signal has been identified, the method proceeds to step 130 comprising determining a signal quality value of the ECG signal based on the identified fiducial point of the ECG signal. In this exemplary embodiment, step 130 comprises sub-steps 132 and 134. Step 132 comprises determining a value of a signal quality indicator based on a portion of the ECG signal associated with the identified fiducial point of the ECG signal. In this example the signal quality indicator comprises at least one of: an amplitude of a QRS complex; an amplitude difference between a QRS complex and a surrounding waveform of the ECG signal; a level of high frequency noise; a level of low frequency noise; a slope of a QRS complex; an area of a QRS complex; and a QRS complex polarity. However, the present invention is not limited these signal quality indicators, and any other value discernible from the ECG signal that may act as a metric for the quality of the ECG signal may act as a signal quality indicator in the present invention.

Step 134 then comprises determining the signal quality value based on the determined value of the signal quality indicator. In this example, this comprises determining a signal quality value describing the suitability of the ECG signal for monitoring the cardiac activity of the subject during a predetermined time period based on the value of the signal quality value. Thus, the method 100 may be repeated to determine a signal quality value of the signal for multiple different portions of the ECG signal each associated with a different period in time. This may be particularly advantageous when the presence of noise in the signal varies significantly over time.

The signal quality value of the present invention may be any qualitative or quantitative value indicating the quality of the ECG signal and its suitability for use in cardiac monitoring. For example, the signal quality value may be a positive number, wherein the magnitude of the determined signal quality value is greater for better quality signals. Alternatively, the signal quality value may be a qualitative description of the ECG signal quality, for example the signal quality value may comprise a categorization of the ECG signal into one of poor quality, medium quality, or high quality.

Although in the method 100 the non-ECG cardiac signal is a PPG signal, this need not be the case in other embodiments. Other suitable non-ECG cardiac signals for use in the present invention may comprise at least one of: an arterial blood pressure signal; a peripheral blood pressure signal; a ballisto-cardiograph signal; and a seismo-cardiograph signal.

Similarly other aspects of the method 100 may be implemented differently in alternative embodiments. The cardiac event detected in step 122 need not comprise a heartbeat corresponding to the systolic phase of a cardiac cycle. The identification of other cardiac events within the non-ECG cardiac signal may equally well be used to identify fiducial points in the ECG signal depending on the particular fiducial points used.

Further, in certain instances, algorithms may be configured to identify corresponding portions of the ECG signal and the non-ECG cardiac signal without requiring exact time calibrations between the signal. This may particularly be the case where the fiducial point corresponds to a portion of the ECG signal that is associated with a time duration significantly longer than the time delay between the non-ECG cardiac signal and the ECG signal. Therefore, in some embodiments, time calibration may be omitted from step 124.

The fiducial point identified in the ECG signal need not comprise a time interval corresponding to a heartbeat of the subject. Alternatively, or in addition, the fiducial point may comprise at least one of an onset time of a heartbeat of the subject; an offset time of a heartbeat of the subject; and a time interval corresponding to a cardiac event. In the method 100 the fiducial point of the ECG signal corresponds exactly to the cardiac event detected in the PPG signal. However, in other embodiments, this need not be the case. Alternatively, the fiducial point of the ECG may correspond to a different cardiac event to that detected in the non-ECG cardiac signal, and its position in the ECG signal may be calculated from a known temporal relationship between the fiducial point and the cardiac event detected in the non-ECG cardiac signal.

Determining the signal quality value need not comprise sub-steps 132 and 134. For example, in an alternative embodiment, the signal quality value may be determined by processing an identified portion of the ECG signal associated with an identified fiducial point of the ECG signal with a machine learning model trained to take segments of ECG signals as input and output a determined signal quality value of the signal. The model may be trained on training data including training segments of ECG signals and associated known signal quality values as annotated by trained healthcare professionals.

The signal quality value determined in the method 100 describes the general quality of the signal over a given time period. In alternative embodiments, the signal quality value may specifically indicate the usefulness of the signal for a particular further processing or diagnostic step. For example, in some embodiments the signal quality values may describe the suitability of the ECG signal for use in arrhythmia detection. Further, the signal quality value need not describe the quality of only a portion of the ECG signal associated with a predetermined time period but instead may describe the quality of the entire ECG signal.

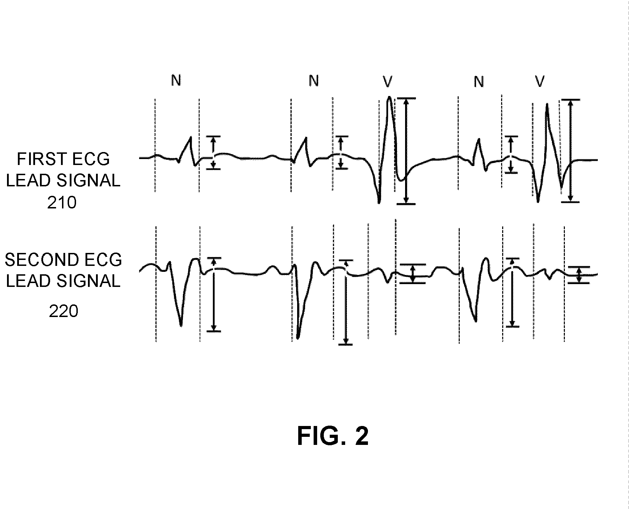

Referring now to Fig. 2 there is depicted a visual representation of an assessment of signal quality based solely on an ECG lead signal. This may be a method of assessing ECG signal quality in contrast to the proposed invention which uses both an ECG signal and a non-ECG signal.

Fig. 2 shows a first ECG lead signal 210 and a second ECG lead signal 220 describing a subject's cardiac activity over a given time period. The signal quality of the first and second ECG signals is assessed by comparing the values of QRS amplitude detected by each of the leads.

In this assessment method, first, the ECG signals are processed to identify the locations of heartbeats in the signal. Due to the nature of the ECG signal, both normal heart beats (N) and premature heartbeats (V) are detected as a result of this process. The regions of the first and second ECG lead signals associated with these detections are indicated by the shaded regions in Fig. 2. Next, the QRS amplitude is calculated for each of the detected heart beats. These values are indicated by the vertical double-headed arrows in Fig. 2.

ECG signal quality for both the first and second ECG lead signals is then assessed based on the calculated QRS amplitude values. Larger QRS amplitude is typically indicative of better quality signal. Therefore, when the ECG signal quality is assessed using the QRS amplitude of all the beats that have been detected in the two ECG signals, the first ECG lead signal 210 is identified as having better signal quality compared to the second ECG lead signal 220, since the average value of the QRS amplitude is greater for the first ECG lead signal.

However, when considering only normal heartbeats, it is clear that the second ECG lead signal 220 is of better quality than the first ECG lead signal 210, since the average value of the QRS amplitude for normal heart beats is greater for in the second ECG lead signal 220 than the first ECG lead signal 210. Typically, the quality of the portion of an ECG signal corresponding to normal heartbeats is more indicative of the usefulness of the signal for monitoring and diagnosis than the portions of the signal corresponding to other cardiac events. Therefore, basing the assessment of signal quality on all the identified heartbeats (including both normal and premature heart beats) may lead to an unreliable assessment of the relative quality of the two signals.

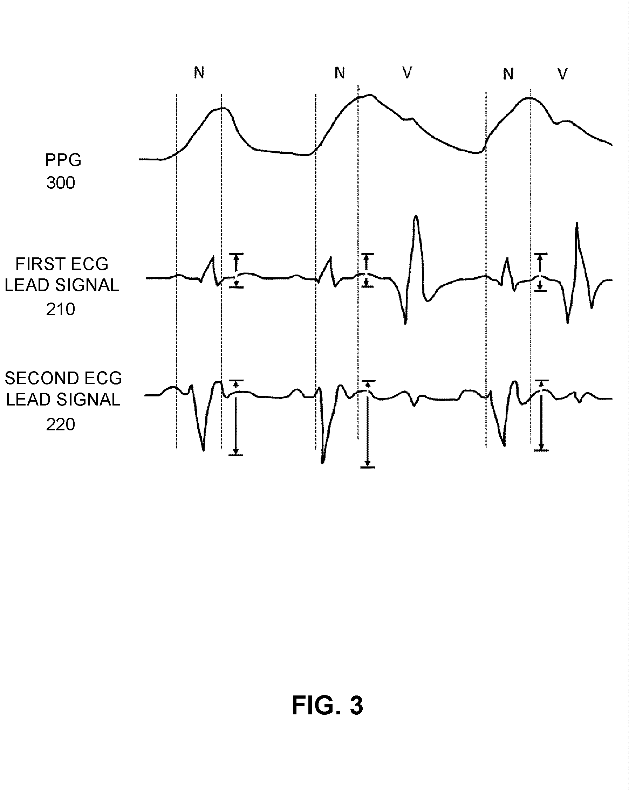

Referring now to Fig. 3, there is depicted a visual representation of an assessment of signal quality according to an embodiment of the proposed invention.

In this example, a PPG signal 300 is obtained alongside the first and second ECG signals. The PPG signal 300 is processed to identify the location of heartbeats within the signal. Since the PPG signal is less sensitive to premature heart beats than the ECG signals, no premature heart beats are detected when using the PPG signal, with only regions of the signal corresponding to normal heart beats being identified.

The assessment of the quality of each of the first and second ECG signals may then be carried out based only on the portions of each ECG signal associated with heartbeats identified in the PPG signal. These regions are indicated by the shaded regions in Fig. 3. When the average QRS amplitude is measured for only these heartbeats, the second ECG lead signal is correctly determined to be of higher quality than the first ECG lead signal. The proposed invention may therefore advantageously lead to a more reliable assessment of signal quality.

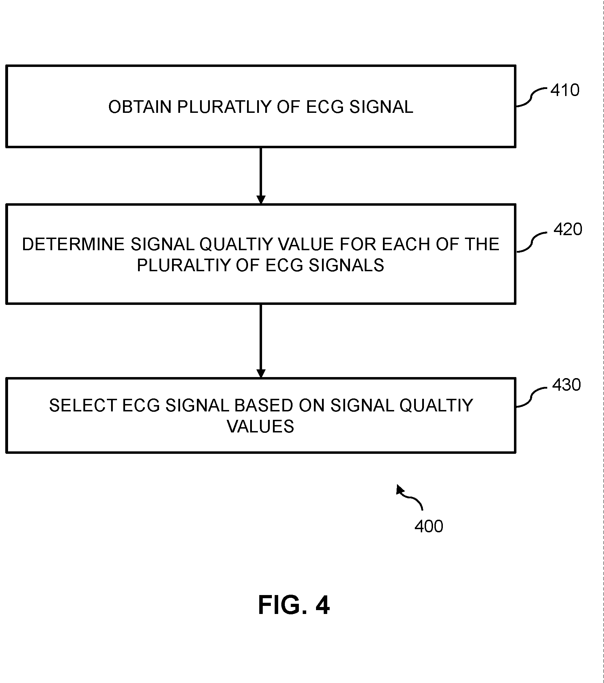

Referring now to Fig. 4 there is depicted a simplified flow diagram of a method 400 for selecting ECG signals for use in monitoring the cardiac activity of a subject according to an aspect of the proposed invention. In the case where the monitoring configuration results in a redundancy of ECG signals for patient monitoring, specific ECG signals may be selected from a plurality of ECG signals based on the determined signal quality value of each of the signals.

The method commences with a step 410 comprising obtaining a plurality of ECG signals for use in monitoring cardiac activity of a subject. Typical ECG monitoring systems comprise multiple electrode sensors and ECG leads that each measure an ECG signal describing the cardiac activity of the subject. The plurality of ECG signals may therefore correspond to a plurality of leads in a single ECG monitoring device. In other embodiments, the plurality of ECG signals may be acquired using multiple ECG sensing devices.

Once the plurality of ECG signals has been obtained, the method then proceeds to a step 420 comprising, for each of the plurality of ECG signals, determining a signal quality value using any of the proposed methods of the present invention. For example, a signal quality value for each of the plurality of ECG signals may be determined using the method 100 described above.

Once the signal quality values have been determined, the method proceeds to a step 430 comprising selecting an ECG signal from the plurality of ECG signals for use in monitoring the subject based on the determined signal quality values of each of the plurality of ECG signals. This selection process may simply comprise selecting the ECG signal of the plurality of ECG signals determined to have the highest signal quality value or may involve comparing the determined signal values to predetermined threshold values. More complicated selection algorithms are also envisioned which utilize the determined signal quality values alongside other known quantities such as physiological parameters of the patient, parameters of the ECG monitoring set up, and details of the further processing steps and diagnosis procedures that are to be carried out using the selected ECG signal(s).

Fig. 4 depicts a method for selecting an ECG signal from a plurality of ECG signals. A similar selection process may be carried out to select suitable portions of a single ECG signal for use in visualization and signal interpretation. In this proposed aspect, the non-ECG cardiac signal may be used to identify a fiducial point in each of a plurality of portions of the ECG signal. A signal quality value for each of the plurality of portions of the ECG signal is then determined based on the identified fiducial point of the portion of the ECG signal. One of the plurality of portions of the ECG signal is then selected from the plurality of portions of the ECG signal for use in a signal interpretation step based on the determined signal quality values. For example, this method may be used to select portions of an ECG signal for automatic arrythmia detection.

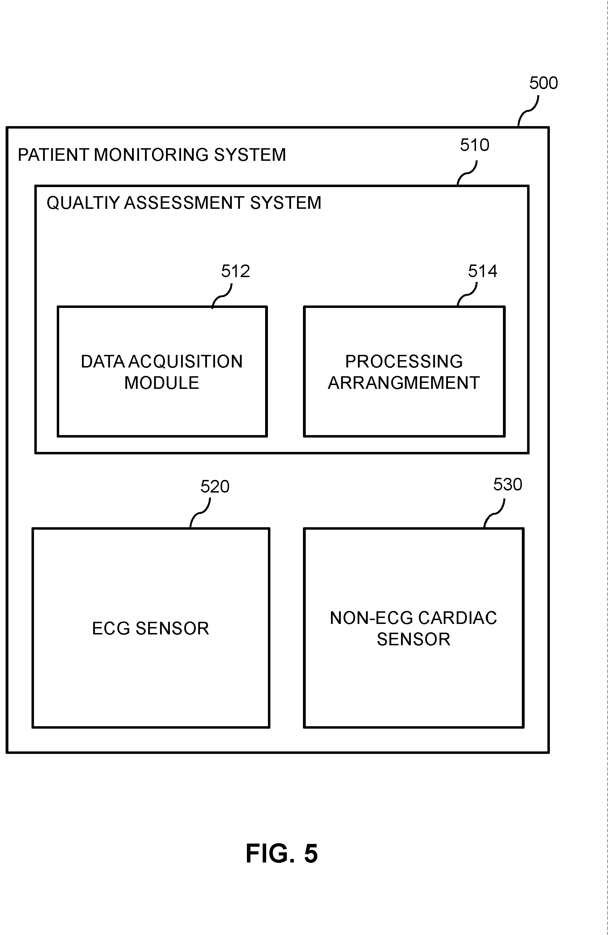

Referring now to Fig. 5 there is depicted a patient monitoring system 500 according to another aspect of the proposed invention.

The patient monitoring system 500 comprises a quality assessment system 510, and ECG sensor 520 and a non-ECG cardiac sensor 530. The patient monitoring system 500 may also include other physiological sensors (not shown) for measuring other clinical parameters of a patient. In this way, the patient monitoring system 500 may comprise a multi-parameter patient monitoring system. The ECG sensor 520 is configured to obtain an ECG signal for monitoring the cardiac activity of a subject. The non-ECG cardiac sensor 530 is configured to acquire a non-ECG cardiac signal for monitoring the cardiac activity of the subject.

The quality assessment system 510 comprises a data acquisition module 512 and a processing arrangement 514. The ECG signal and the non-ECG cardiac signal acquired by the ECG sensor 520 and the non-ECG cardiac sensor 530 are passed to the data acquisition module 512 of the quality assessment system 510. The processing arrangement 514 is then configured to identify a fiducial point of the ECG signal based on the non-ECG cardiac signal; and determine a signal quality value of the ECG signal based on the identified fiducial point of the ECG signal.

The patient monitoring system 500 may further comprise other components not shown in Fig. 5, such as an ECG selection module configured to select an ECG signal from a plurality of ECG signals acquired by the ECG sensor for use in monitoring the cardiac activity of the subject, based on a signal quality value of each of the plurality of ECG signals as determined by the ECG quality assessment system 510. Other optional components of the patient monitoring system 500 include an output interface configured to display visual representations of the ECG signal and the non-ECG signal to a healthcare professional.

The concepts of the proposed invention may therefore be integrated into a multi-parameter patient monitoring system to provide improved cardiac monitoring.

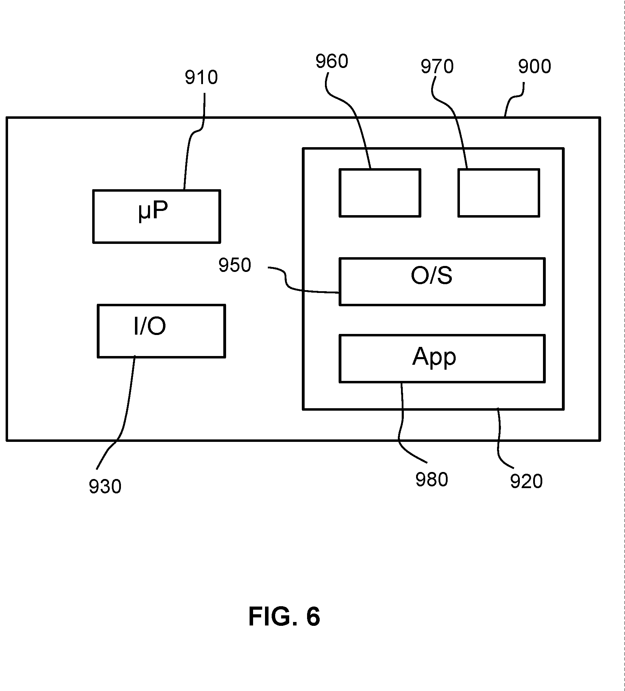

Fig. 6 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g., connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920, and one or more I/O devices 930 that are communicatively coupled via a local interface (not shown). The local interface can be, for example but not limited to, one or more buses or other wired or wireless connections, as is known in the art. The local interface may have additional elements such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s) and system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program code configured to cause one or more physical computing devices to carry out a quality assessment method as described above when the program is run one the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPAGs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module segment, or portion of instruction, which comprises one or more executable instructions for implementing the specified logical function(s). In some alternative implementations, the functions noted in the block may occur out of the order noted in the Figures. For example, two blocks shown in succession may, in face be executed substantially concurrently, or the blocks may sometimes be executed in reverse order, depending on the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A computer-implemented method for assessing the quality of an ECG signal, the method comprising:
obtaining an ECG signal and a non-ECG cardiac signal for use in monitoring the cardiac activity of a subject;
identifying a fiducial point of the ECG signal based on the non-ECG cardiac signal; and
determining a signal quality value of the ECG signal based on the identified fiducial point of the ECG signal.

2. The method of claim 1, wherein identifying a fiducial point of the ECG signal based on the non-ECG cardiac signal comprises:
processing (122) the non-ECG cardiac signal with a cardiac event detection model to identify a portion of the non-ECG signal associated with a cardiac event of the subject;
identifying (124) a portion of the ECG signal that corresponds to the identified portion of the non-ECG cardiac signal; and
identifying (126) the fiducial point of the ECG signal based on the identified portion of the ECG signal.

3. The method of claim 2, wherein the cardiac event of the subject comprises a heartbeat corresponding to the systolic phase of a cardiac cycle of the subject.

4. The method of any of claims 2 and 3, wherein identifying a portion of the ECG signal comprises performing a time calibration process to synchronize the ECG signal and the non-ECG cardiac signal.

5. The method of any preceding claim, wherein the fiducial point comprises at least one of:
a time interval corresponding to a heartbeat of the subject;
an onset time of a heartbeat of the subject;
an offset time of a heartbeat of the subject ; and
a time interval corresponding to a cardiac event.

6. The method of claim 1, wherein determining a signal quality value of the ECG signal comprises:
determining (132) a value of a signal quality indicator based on a portion of the ECG signal associated with the identified fiducial point of the ECG signal; and
determining (134) the signal quality value based on the value of the signal quality indicator.

7. The method of claim 6, wherein the signal quality indicator comprises at least one of:
an amplitude of a QRS complex;
an amplitude difference between a QRS complex and a surrounding waveform of the ECG signal;
a level of high frequency noise;
a level of low frequency noise;
a slope of a QRS complex;
an area of a QRS complex; and
a QRS complex polarity.

8. The method of any preceding claim wherein the non-ECG cardiac signal comprises at least one of:
a PPG signal;
an arterial blood pressure signal;
a peripheral blood pressure signal;
a ballisto-cardiograph signal; and
a seismo-cardiograph signal.

9. The method of any preceding claim, wherein the signal quality value describes the suitability of the ECG signal for use in arrhythmia detection.

10. The method of any preceding claim, wherein the signal quality value describes the suitability of the ECG signal for monitoring the cardiac activity of the subject during a predetermined time period.

11. A method (400) for selecting ECG signals for use in monitoring the cardiac activity of a subject, the method comprising:
obtaining (410) a plurality of ECG signals for use in monitoring the cardiac activity of a subject;
for each of the plurality of ECG signals determining (420) a signal quality value using the method of any of claims 1-10; and
selecting (430) an ECG signal from the plurality of ECG signal for use in monitoring the subject based on the determined signal quality values of each of the plurality of ECG signals.

12. A computer program comprising computer program code means adapted, when said computer program is run on a computer, to implement the method of any of claims 1-11.

13. An ECG quality assessment system (510) comprising:
a data acquisition module (512) configured to obtain an ECG signal and a non-ECG cardiac signal for use in monitoring the cardiac activity of a subject; and
a processing arrangement (514) configured to:
identify a fiducial point of the ECG signal based on the non-ECG cardiac signal; and
determine a signal quality value of the ECG signal based on the identified fiducial point of the ECG signal.

14. A patient monitoring system (500) comprising:
an ECG sensor (520) configured to acquire an ECG signal;
a non-ECG cardiac sensor (530) configured to acquire a non-ECG cardiac signal; and
the ECG quality assessment system (510) of claim 13.

15. The patient monitoring system of claim 14, wherein the non-ECG cardiac sensor comprises at least one of:
a PPG sensor;
an arterial blood pressure sensor;
a peripheral blood pressure sensor;
a ballisto-cardiogram sensor; and
a seismo-cardiogram sensor.
